# FASCICULE DE BREVET EUROPEEN

(11) **EP 4 486 399 B1**
(45) Date de publication et mention de la délivrance du brevet: **06.05.2026**
(21) Numéro de dépôt: 23708238.3
(22) Date de dépôt: 03.03.2023
(51) Int. Cl.: A61L 2/00, A61J 1/10, A61K 41/17, A61M 1/36, A61M 1/02

(54) **SYSTÈME À POCHES POUR LE TRAITEMENT PAR IRRADIATION ÉLECTROMAGNÉTIQUE D'UN FLUIDE BIOLOGIQUE**
BEUTELSYSTEM ZUR BEHANDLUNG EINER BIOLOGISCHEN FLÜSSIGKEIT DURCH ELEKTROMAGNETISCHE BESTRAHLUNG
POUCH SYSTEM FOR TREATMENT OF A BIOLOGICAL FLUID BY ELECTROMAGNETIC IRRADIATION

(30) Priorité: 04.03.2022 FR 2201902
(43) Date de publication de la demande: 08.01.2025
(73) Titulaire: Maco Pharma, 59420 Mouvaux (FR)
(72) Inventeur: BIRCK, Cécile, 59420 Mouvaux (FR); JOURJON, Charly, 59420 Mouvaux (FR)
(74) Mandataire: Sayettat, Julien Christian
(86) Numéro de dépôt international: PCT/EP2023/055504
(87) Numéro de publication internationale: WO 2023/166209

(56) Documents cités:
- EP-B1- 0 035 065
- WO-A2-2021/231650
- FR-A1- 2 965 812
- JP-B2- 6 340 611
- US-A1- 2013 074 454
- US-A1- 2019 230 919

## Description

L'invention concerne un système à poches pour le traitement par irradiation électromagnétique d'un fluide biologique, ainsi qu'un ensemble comprenant ledit système à poches et un emballage.

Elle s'applique au domaine médical et biomédical, en particulier au domaine de la transfusion sanguine et encore plus particulièrement au domaine du traitement du sang.

Dans le cadre de la transfusion, le sang est d'abord collecté d'un donneur puis séparé par centrifugation en différents produits sanguins tels que les concentrés de globules rouges, le plasma et les concentrés de plaquettes. Afin d'améliorer la qualité et la sécurité de ces différents produits sanguins, ceux-ci peuvent subir différents traitements tels que l'élimination des leucocytes et/ou la réduction des pathogènes.

Le document WO 2007/076834 décrit un procédé pour réduire les pathogènes tels que les bactéries, les virus et/ou les leucocytes des concentrés plaquettaires. Dans ce procédé, on irradie avec un rayonnement UV et sous agitation, une poche contenant un concentré plaquettaire. Ce procédé possède l'avantage par rapport à d'autres procédés d'inactivation de pathogènes tels que les procédés commerciaux Intercept (Cerus) et Mirasol (TerumoBCT), de ne pas utiliser d'agent photosensible tel que l'amotosalène ou la riboflavine.

Dans ces procédés utilisant la lumière, et notamment en l'absence d'agent photosensible, il est important de s'assurer que le produit sanguin à traiter reçoive la dose nécessaire et suffisante pour réduire le taux de pathogènes à un niveau acceptable, sans détériorer les propriétés biologiques du produit sanguin.

Le document WO 2008/034476 décrit un système à poches adapté pour mettre en œuvre le procédé de réduction des pathogènes par rayonnement UV décrit dans le document WO 2007/076834. Le système à poches comprend essentiellement une poche d'irradiation réalisée en copolymère d'éthylène - acétate de vinyle (EVA) reliée à une poche de conservation des concentrés plaquettaires réalisée en polychlorure de vinyle (PVC) plastifié. Le système à poches comprend également des tubulures réalisées en PVC plastifié.

Dans ce document, il est indiqué que l'absorption de la lumière UV par l'EVA est influencée par le degré de polymérisation et de réticulation de l'EVA.

D'autres facteurs intrinsèques influent sur la transmittance UV d'une feuille en EVA, tels que la proportion relative des motifs d'éthylène et d'acétate de vinyle, l'épaisseur du film ou son état de surface. Parmi les facteurs extrinsèques ayant un impact sur la transmittance UV, on note la chaleur, l'humidité, la présence d'oxygène et une exposition aux rayons UV.

Après de nombreuses recherches, la demanderesse a identifié un autre facteur jusquelà non décrit, impactant la transmittance UV d'une feuille en EVA.

La demanderesse a en effet mis en évidence que la transmittance UV d'une poche en EVA se détériore par la migration de certains plastifiants du PVC des autres éléments du système à poches ainsi que par la migration de certaines substances de l'emballage dans lequel est confiné le système à poches.

En effet, les systèmes à poches tels que celui décrit dans le document WO 2008/034476 comprennent des éléments tels que des connecteurs ou des tubulures, réalisés en PVC plastifié par le phtalate de di-2-éthylhéxyle (DEHP, di-2-ethylhexyl phthalate).

Le DEHP est en outre connu pour être un perturbateur endocrinien avec des effets possibles sur le système reproductif, hormonal et immunitaire. Il est également potentiellement cancérogène. Ainsi, il est recommandé d'éliminer le DEHP des dispositifs médicaux tels que les systèmes de poches pour le traitement du sang.

Des systèmes à poches sans DEHP pour le traitement du sang sont connus. Par exemple, le document JP2003-171288A décrit un système à poches comprenant un filtre à éliminer les leucocytes du sang total et une pluralité de poches destinées à contenir les composants sanguins, connectées audit filtre. L'une au moins des poches contient un plastifiant de type citrate, les autres poches étant réalisées en PVC plastifié par un ester de citrate ou un ester de trimellitate.

Le document EP 2 731 425 B1 décrit une poche sans plastifiant de type phtalate destinée à conserver les produits de globules rouges. La poche est réalisée dans un matériau polymère plastifié par l'ester de diisononyle de l'acide 1,2-cyclohexanedicarboxylique (DINCH) et contient une solution additive particulière.

Les systèmes à poches en PVC de ces deux documents ne sont pas adaptés à l'irradiation électromagnétique des composants sanguins et le choix du plastifiant est motivé par la réduction de l'hémolyse des globules rouges dans des poches sans DEHP.

Plutôt que de supprimer le DEHP des dispositifs médicaux, le document FR 2965812 propose de supprimer la migration du DEHP ou autre composé plastifiant d'un objet en PVC plastifié tel qu'une tubulure, en enduisant l'objet d'une couche d'oxyde métallique comme le dioxyde de titane.

D'autre part, il est connu du document EP 1 972 354, un système à poches pour la séparation et la réduction des pathogènes d'un fluide biologique à l'aide d'un agent photosensible activable par rayonnement ultraviolet, dans lequel la ou les poches contenant l'agent photosensible sont arrangées dans une surenveloppe opaque amovible. Cette surenveloppe permet d'éviter l'activation précoce de l'agent photosensible par la lumière ambiante.

Enfin, Le document US 2019/0230919 décrit des kits pour réduire le niveau d'oxygène dans le sang ou les composants sanguins afin d'en améliorer la conservation. Ces kits comprennent essentiellement un récipient externe fermé et imperméable à l'oxygène ; une poche souple interne destinée à recevoir le sang ou les composants sanguins ; et un absorbeur d'oxygène placé dans ledit récipient externe. La poche interne est réalisée dans un matériau perméable à l'oxygène tel que le polyfluorure de vinylidène, le silicone ou le polyuréthane. Le document WO 2021/231650 décrit des kits similaires dans lesquels la poche interne est notamment réalisée en polychlorure de vinyle plastifié par le phtalate de di-2-éthylhéxyle (DEHP), l'ester de diisononyle de l'acide 1,2-cyclohexanedicarboxylique (DINCH) ou le citrate de butyryle et de trihéxyle (BTHC). Du fait du récipient externe fermé enfermant la poche interne, ces kits ne sont pas adaptés au traitement du sang ou des composants sanguins par irradiation électromagnétique.

Afin de préserver la transmittance d'une poche d'irradiation d'un système à poches, l'invention propose, selon un premier aspect, un système à poches pour le traitement par irradiation électromagnétique d'un fluide biologique, ledit système à poches comprenant au moins un élément constitué d'une poche d'irradiation destinée à contenir le fluide biologique à irradier, ladite poche d'irradiation étant réalisée dans un matériau perméable audit rayonnement électromagnétique, ledit système comprenant un ou plusieurs autres éléments choisi dans le groupe constitué par au moins une poche de stockage et une tubulure, au moins un desdits autres éléments étant réalisé dans un matériau polymère formulé avec un plastifiant et au moins un film protecteur formant moyen barrière au plastifiant et recouvrant ladite poche d'irradiation, ledit film protecteur étant réalisé dans un matériau polymère barrière aux gaz ayant une perméabilité à l'oxygène inférieure ou égale à 100 cm³/m²/24 h à une température de 23°C et une humidité relative de 50 %.

Selon un deuxième aspect, l'invention porte sur un ensemble pour le traitement par irradiation électromagnétique d'un fluide biologique comprenant un emballage et un système à poches selon le premier aspect de l'invention, ledit système à poches étant confiné de façon stérile dans ledit emballage.

Selon un troisième aspect, l'invention concerne l'utilisation d'un film réalisé dans un matériau polymère barrière aux gaz ayant une perméabilité à l'oxygène inférieure ou égale à 100 cm³ /m²/24 h à une température de 23°C et une humidité relative de 50 % comme moyen barrière aux plastifiants relargables du polychlorure de vinyle.

D'autres objets et avantages apparaîtront au cours de la description qui suit.
[Fig.1] représente une vue schématique d'un ensemble selon une réalisation de l'invention comprenant un emballage stérile dans lequel est disposé un système à poches pour l'irradiation par rayonnement UV d'un produit sanguin.
[Fig.2] représente le taux de transmittance UV d'une feuille réalisée en EVA entre 0 et 1200 jours, au-dessous de laquelle est disposée une autre feuille réalisée en EVA et au-dessus de laquelle sont superposées une ou deux feuilles réalisées en PVC plastifié par le DEHP, le DEHT, le TOTM ou le DINCH (face A).
[Fig.3] représente le taux de transmittance UV d'une feuille réalisée en EVA entre 0 et 1200 jours, au-dessus de laquelle sont superposées une autre feuille réalisée en EVA et une ou deux feuilles réalisées en PVC plastifié par le DEHP, le DEHT, le TOTM ou le DINCH (face B).

L'invention concerne un système à poches et un ensemble pour le traitement par irradiation électromagnétique d'un fluide biologique.

Le fluide biologique est notamment le sang total ou un produit sanguin obtenu par filtration et/ou centrifugation du sang total tel qu'un plasma, un plasma riche en plaquettes, un concentré de globules rouges ou un concentré de plaquettes.

Afin notamment de réduire les pathogènes tels que les virus, les bactéries et/ou les leucocytes du sang ou d'un produit sanguin, il est connu d'appliquer sur le sang ou le produit sanguin, en présence ou non d'un agent photosensible, un rayonnement électromagnétique.

Par rayonnement électromagnétique, on entend un rayonnement non ionisant ayant des longueurs d'onde allant de 10 nm à 10 µm, c'est-à-dire un rayonnement visible, ultraviolet (UV) et/ou infrarouge. En particulier, le rayonnement électromagnétique est un rayonnement visible ayant des longueurs d'onde allant de 340 nm à 800 nm et/ou un rayonnement UV ayant des longueurs d'onde allant de 200 à 340 nm. Plus particulièrement, le rayonnement est un rayonnement UV-C ayant des longueurs d'ondes allant de 200 à 280 nm. Encore plus particulièrement, le rayonnement UV-C possède une longueur d'onde de l'ordre de 254 nm.

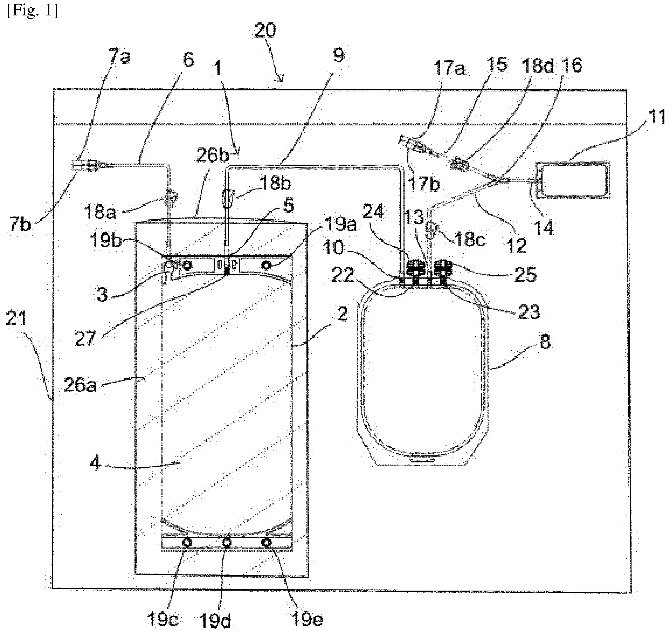

Selon la [Fig.1], l'ensemble 20 pour le traitement par irradiation magnétique comprend un emballage 21 et un système à poches 1 confiné de façon stérile dans ledit emballage 21.

L'emballage 21 est apte à permettre la stérilisation du système à poches 1 dans lequel il est confiné.

Dans une réalisation, l'emballage est un emballage adapté à la stérilisation par des gaz, tel que l'oxyde d'éthylène.

Selon une réalisation, l'emballage est réalisé dans un ou des matériaux formulés sans phtalate ni téréphtalate. Par exemple, l'emballage 21 est formé par assemblage d'une feuille poreuse en non-tissé et d'une feuille en film transparent. La feuille poreuse laisse passer le gaz stérilisant à l'intérieur de l'emballage tout en empêchant le passage de microbes dans ledit emballage. La feuille poreuse est par exemple réalisée en non-tissé de cellulose tel que le papier ou en non-tissé synthétique tel qu'en fibres de polyéthylène haute densité (Tyvek^{®}). La feuille en film transparent est par exemple réalisée en polymère transparent tel que le polyéthylène haute densité, le polyéthylène basse densité, le polypropylène et/ou le polyester. La feuille poreuse en non-tissé et la feuille en film transparent sont par exemple assemblées par soudure.

Selon la [Fig.1], le système à poches pour l'irradiation par rayonnement électromagnétique d'un fluide biologique comprend au moins un élément constitué d'une poche d'irradiation 2 destinée à contenir le fluide biologique à irradier. La poche d'irradiation est réalisée dans un matériau perméable audit rayonnement électromagnétique.

Selon une réalisation, l'irradiation par rayonnement électromagnétique est une irradiation par rayonnement UV, notamment rayonnement UV-C et la poche d'irradiation est réalisée dans un matériau perméable à un rayonnement UV, notamment un rayonnement UV-C.

Le fluide biologique à traiter est notamment le sang ou un produit sanguin tel qu'un concentré plaquettaire.

Par exemple, la poche d'irradiation 2 est réalisée en EVA. En alternative, la poche d'irradiation 2 est réalisée dans un matériau polymère perméable à un rayonnement UV tel que le polymethylpentène, le poly-chloro-trifluoroéthylène, le perfluoroalkoxy, l'ethylène propylene fluoré, l'éthylène tétrafluoroéthylène, l'éthylène chlorotrifluoroéthylène ou le polyfluorure de vinylidène. Le matériau de la poche d'irradiation est formulé sans phtalate ni téréphtalate.

Par phtalate ou téréphtalate, on entend un dérivé de l'acide phtalique ou téréphtalique, tel qu'un ester de l'acide phtalique ou téréphtalique.

Un matériau formulé sans phtalate ni téréphtalate est un matériau dans lequel aucun phtalate ni téréphtalate en tant que plastifiant n'a été ajouté dans la composition du matériau.

Le matériau formulé sans phtalate ou téréphtalate peut contenir des traces résiduelles de phtalate ou téréphtalate, c'est-à-dire une teneur inférieure à 1000 ppm, liées par exemple à une contamination lors du procédé de fabrication du matériau ou du système à poches ou lors de son emballage.

En particulier, la poche d'irradiation 2 est formée à partir d'une gaine ou par assemblage de deux feuilles perméables à un rayonnement UV, la gaine ou chaque feuille possédant une transmittance UV-C supérieure à 60%, notamment supérieure à 65%. Encore plus particulièrement, la transmittance UV-C de la gaine ou de chacune des feuilles est inférieure à 90%, notamment inférieure à 85%.

Par transmittance UV, on entend le rapport en pourcentage entre l'intensité du rayonnement transmis à l'intensité incidente. La transmittance est déterminée par spectrophotométrie.

Selon une réalisation particulière, la poche d'irradiation 2 est exempte d'agent photosensible. Autrement dit, la poche d'irradiation 2 ne contient pas d'agent photosensible tel que la riboflavine, un dérivé de psoralène ou un dérivé de phénothiazine

Selon la [Fig.1], la poche d'irradiation 2 comprend un premier orifice d'accès 3 en communication fluidique avec le volume intérieur 4 de la poche d'irradiation 2. La poche d'irradiation 2 comprend en outre un deuxième orifice d'accès 5 en communication fluidique avec le volume intérieur 4 de la poche d'irradiation 2.

La poche d'irradiation 2 est en en communication fluidique avec une première tubulure 6 par l'intermédiaire du premier orifice d'accès 3. La première tubulure 6 est pourvue à son extrémité de deux évents 7a,7b comprenant chacun une membrane filtrante laissant passer l'air et formant une barrière stérile. Ces évents permettent la stérilisation par gaz du système à poches 1.

La poche d'irradiation 2 comprend en outre des ouvertures 19a,19b,19c,19d,19e pratiquées dans un ou des bords périphériques de la poche d'irradiation 2 permettant de suspendre et/ou maintenir ladite poche d'irradiation 2.

Le système à poches 1 comprend en outre une poche de stockage 8 destinée à recueillir et stocker le composant sanguin traité. Cette poche de stockage 8 est en communication fluidique avec la poche d'irradiation 2 par l'intermédiaire de la deuxième tubulure 9 connectée à une extrémité à un deuxième orifice d'accès 5 de la poche d'irradiation 2 et à l'autre extrémité à un orifice d'accès 10 de la poche de stockage 8. La poche de stockage 8 comprend en outre deux orifices de sortie 22,23, chacun fermés par un bouchon sécable 24,25 comportant des ailettes.

Un bouchon amovible 27 est disposé dans le deuxième orifice d'accès 5 de la poche d'irradiation 2, fermant la communication fluidique entre le volume intérieur 4 de la poche d'irradiation 2 et la deuxième tubulure 9 menant à la poche de stockage 8. Le bouchon amovible 27 peut glisser hors du deuxième orifice d'accès 5, dans le volume intérieur 4 du récipient 2 afin de permettre la communication fluidique entre la poche d'irradiation 2 et la poche de stockage 8.

Le système à poches 1 comprend en outre une poche d'échantillonnage 11 en communication fluidique avec la poche de stockage 8 par l'intermédiaire d'une troisième tubulure 12 reliée à l'une de ses extrémités à un deuxième orifice d'accès 13 de la poche de stockage 8 et à son autre extrémité, à un orifice d'accès 14 de la poche d'échantillonnage 11.

Une quatrième tubulure 15 est connectée à l'une de ses extrémités à la troisième tubulure 12 par l'intermédiaire d'un connecteur trois voies 16. L'autre extrémité de cette quatrième tubulure 15 est pourvue de deux évents 17a,17b comprenant chacun une membrane filtrante laissant passer l'air et formant une barrière stérile.

Chacune des tubulures 6,9,12,15 est pourvue d'une pince 18a,18b,18c,18d qui permet le contrôle sélectif d'ouverture et fermeture de l'écoulement des liquides dans chacune de ces tubulures.

Le système à poches 1 comprend avantageusement au moins un film protecteur 26a recouvrant ladite poche d'irradiation. Le film protecteur est une feuille très mince de matière plastique. Il est réalisé dans un matériau polymère formulé sans phtalate ni téréphtalate. Le film protecteur 26a est superposé à la poche d'irradiation en couvrant entièrement sa surface. Le film protecteur 26a est par exemple disposé entre la poche d'irradiation 2 et la feuille poreuse, notamment réalisée en papier laqué, de l'emballage 21.

Le film protecteur a pour fonction de protéger la poche d'irradiation 2 des substances pouvant nuire à la transmittance, en particulier la transmittance UV, et encore plus particulièrement la transmittance UV-C, de ladite poche d'irradiation. La poche d'irradiation 2 est découverte de ce film protecteur au moment de son traitement par rayonnement électromagnétique.

Ce film protecteur forme ainsi un moyen barrière aux substances relargables des autres éléments du système à poches, et qui nuisent à la transmittance de la poche d'irradiation. Par substance relargable, on entend une substance qui migre d'un élément du système à poches ou de son emballage. Les substances relargables sont par exemple les plastifiants du PVC, tel que les phtalates ou téréphtalates, les esters de l'acide cyclohexanedicarboxylique ou les esters de citrate. Autrement dit, le film protecteur forme un moyen barrière au plastifiant d'un matériau polymère tel que le PVC.

En effet, dans son emballage 21, le système à poches 1 est plié de sorte à notamment disposer la poche de stockage 8 sur la poche d'irradiation 2. Dans cette configuration, sans film protecteur, les plastifiants relargables du système à poches migrent vers la poche d'irradiation 2. La présence de ces substances relargables sur la poche d'irradiation détériore ses propriétés optiques, en particulier sa transmittance UV, encore plus particulièrement sa transmittance UV-C.

Ce film protecteur protège également la poche d'irradiation des substances pouvant nuire à la transmittance de la poche d'irradiation et qui se trouvent dans l'environnement. En effet, les phtalates et notamment le DEHP, sont présents partout dans l'environnement et sources potentielles de contamination de la poche d'irradiation.

Selon une réalisation, un film protecteur réalisé dans un matériau polymère barrière aux gaz ayant une perméabilité à l'oxygène inférieure ou égale à 100 cm³/m²/24 h à une température de 23°C et une humidité relative de 50 % est utilisé comme moyen barrière aux plastifiants relargables du polychlorure de vinyle, tels que le DEHP ou le DEHT. Notamment, le matériau du film protecteur est en outre un matériau polymère barrière à la vapeur d'eau présentant une perméabilité à la vapeur d'eau inférieure ou égale à 10 g/m²/24 h à une température de 23°C et une humidité relative de 50 %.

Ainsi, le film protecteur 26a est réalisé dans un matériau polymère barrière aux gaz ayant une perméabilité à l'oxygène inférieure ou égale à 100 cm³/m²/24 h, plus particulièrement inférieure ou égale à 90, à une température de 23°C et une humidité relative de 50 %. La perméabilité à l'oxygène est mesurée conformément à la norme ASTM D 3985.

En particulier, le matériau polymère du film protecteur 26a est un matériau polymère barrière à la vapeur d'eau présentant une perméabilité à la vapeur d'eau inférieure ou égale à 10 g/m²/24 h, plus particulièrement inférieure à 5 g/m²/24 h à une température de 23°C et une humidité relative de 50 %. %. La perméabilité à la vapeur d'eau est mesurée conformément à la norme ASTM F 1249.

En particulier, le film protecteur comprend au moins une couche réalisée en polyester ou polypropylène. Par exemple, le film protecteur comprend une feuille multicouches composite comprenant une couche réalisée en polyester et une couche réalisée en polypropylène. En alternative, le film protecteur comprend une feuille multicouches composite comprenant deux couches réalisées en polyester ou deux couches réalisées en polypropylène.

Avantageusement, le film protecteur est transparent, permettant à l'utilisateur de voir la poche d'irradiation 2.

L'épaisseur du film protecteur est comprise entre 40 et 70 µm, en particulier entre 45 et 65 µm.

Selon une réalisation, le système à poches comprend deux films protecteurs 26a, 26b agencés de part et d'autre de la poche d'irradiation 2. La poche d'irradiation 2 est ainsi intercalée entre les deux films protecteurs 26a,26b jusqu'au moment de son traitement par rayonnement électromagnétique. En alternative, le film protecteur est plié sur luimême de sorte à former un feuillet dans lequel la poche d'irradiation 2 est intercalée.

Selon une autre réalisation, les deux films protecteurs 26a,26b forment ensemble un manchon ayant au moins une extrémité ouverte. L'extrémité ouverte du manchon permet à l'utilisateur de retirer facilement ce manchon de la poche d'irradiation 2 par glissement juste avant le traitement du fluide biologique contenu dans la poche d'irradiation. La poche d'irradiation 2 est disposée dans ce manchon de sorte à l'envelopper. Le manchon entoure la poche d'irradiation 2 pour la protéger de la migration de plastifiants ou autres substances relargables nuisant à la transmittance de la poche d'irradiation 2.

Par exemple, le manchon est réalisé en superposant les deux films protecteurs 26a,26b, et en soudant entre eux deux côtés opposés et un côté transversal.

Dans cette réalisation, la poche d'irradiation 2 est protégée des substances relargables du système à poches 1 et de l'emballage 21.

Afin de s'assurer de la préservation des propriétés optiques de la poche d'irradiation 2 du système à poches 1, outre la poche d'irradiation 2, le ou les autres éléments du système à poches sont réalisés dans un ou des matériaux formulés sans phtalate ni téréphtalate.

Selon une réalisation, chacun desdits autres éléments est réalisé dans un ou des matériaux choisis dans le groupe constitué par un matériau autre que le polychlorure de vinyle plastifié et un matériau polymère formulé avec au moins un plastifiant autre qu'un phtalate tel que le phtalate de di-2-éthylhéxyle ou un téréphtalate tel que le téréphtalate de di-2-éthylhéxyle.

En effet, il a été identifié par la demanderesse que les esters de phtalate et téréphtalate tels que le phtalate de di-2-éthylhéxyle (DEHP) ou le téréphtalate de di-2-éthylhéxyle (DEHT) qui migrent vers la poche d'irradiation 2 abaissent la transmittance UV, plus particulièrement la transmittance UV-C, de la poche d'irradiation 2.

Ainsi, chacun des autres éléments du système à poches 1 est avantageusement réalisé dans un ou des matériaux autre que le polychlorure de vinyle plastifié par le phtalate de di-2-éthylhéxyle ou le téréphtalate de di-2-éthylhéxyle.

Avantageusement et pour les mêmes raisons, l'emballage dans lequel est confiné le système à poches est réalisé dans un ou des matériaux formulés sans phtalate ni téréphtalate, en particulier sans phtalate de di-2-éthylhéxyle (DEHP) ni téréphtalate de di-2-éthylhéxyle (DEHT).

Dans une réalisation particulière, chacun des autres éléments du système à poches est réalisé soit en polychlorure de vinyle plastifié par un plastifiant choisi dans le groupe constitué par un ester d'acide cyclohexanedicarboxylique, un ester de citrate, un ester de trimellitate ou un mélange de ceux-ci, soit en un matériau autre que le polychlorure de vinyle plastifié.

Plus particulièrement encore, chacun des autres éléments du système à poches est réalisé soit en polychlorure de vinyle plastifié par un plastifiant choisi dans le groupe constitué par le diisononyle de l'acide 1,2-cyclohexanedicarboxylique, le citrate de butyryle et de trihéxyle, le trimellitate de tri-2-éthylhéxyle ou un mélange de ceux-ci, soit en un matériau autre que le polychlorure de vinyle plastifié.

Les autres éléments du système à poches comprennent un ou plusieurs éléments choisis dans le groupe constitué par une poche de stockage 8, une poche d'échantillonnage 11, une tubulure 6,9,12,15, un connecteur 16, une pince 18a,18b, 18c, 18d, un évent 7a, 7b, 17a, 17b et leurs combinaisons.

Les éléments rigides du système à poches, par exemple les pinces et les évents sont réalisés dans un matériau autre que le polychlorure de vinyle plastifié, tel que le polycarbonate, le polyester, le polyéthylène et/ou le polypropylène.

Par exemple, les pinces 18a,18b,18c,18d sont réalisées en polycarbonate. Les évents 7a,7b,17a,17b sont réalisés en polypropylène.

Les éléments souples du système à poches, autre que la poche d'irradiation 2, sont réalisés en polychlorure de vinyle plastifié par au moins un plastifiant choisi dans le groupe constitué par un ester d'acide cyclohexanedicarboxylique, un ester de citrate, un ester de trimellitate et un mélange de ceux-ci.

La poche de stockage 8 est réalisée dans un matériau perméable aux gaz pour permettre le stockage pendant au moins 3 à 7 jours d'un concentré de plaquettes. Par exemple, la poche de stockage 8 est réalisée en PVC plastifié par le trimellitate de tris(éthylhéxyle) (ou tri-octyl trimellitate, TOTM) ou le citrate de butyryle et de trihéxyle (BTHC).

Par exemple, la poche d'échantillonnage 11 est réalisée en PVC plastifié par l'ester de diisononyle de l'acide 1,2-cyclohexanedicarboxylique (DINCH).

Par exemple, les tubulures 6, 9, 12, 15 et connecteur 16 sont réalisés en PVC plastifié par le DINCH.

Les composants des poches d'irradiation 2, de stockage 8 et échantillonnage 11 tels que les orifices d'accès 3,5,10,13,14, les orifices de sorties 22,23 et les bouchons 24,25,27 sont également avantageusement réalisés dans un ou des matériaux formulés sans phtalate ni téréphtalate.

En lien avec la réalisation de l'ensemble 20 de la [Fig.1], on décrit maintenant un procédé pour mettre en œuvre le système à poches 1 et son emballage 21.

L'emballage 21 dans lequel est confiné le système à poches 1 est ouvert avant d'en sortir le système à poches 1.

Une poche source (non représentée) contenant un fluide biologique, par exemple un concentré plaquettaire issu d'aphérèse ou un mélange de concentrés plaquettaires obtenus à partir de couches leuco-plaquettaires est connectée stérilement au système à poches 1 par l'intermédiaire de la première tubulure 6, à l'aide d'un appareil de connexion stérile de type TSCD-II (Terumo).

Le fluide biologique de la poche source est ensuite transféré par gravité dans le volume intérieur 4 de la poche d'irradiation 2 par l'intermédiaire du premier orifice d'accès 3.

Après le transfert, l'air contenu dans le volume intérieur 4 de la poche d'irradiation 2 est chassé la poche d'irradiation 2 par le premier orifice d'accès 3 en exerçant une pression sur la poche d'irradiation. Une fois l'air chassé, le premier orifice d'accès 3 est fermé par soudure.

Ces opérations de purge d'air et soudure sont réalisés avantageusement à l'aide de l'appareil à souder décrit dans le document WO2022/029040.

La première tubulure 6 est soudée et coupée afin de séparer la poche source contenant initialement le fluide biologique, du système à poches 1.

Ensuite, la poche d'irradiation 2 est sortie du manchon constitué des deux feuilles 26a,26b. En variante, ce manchon est dégagé de la poche d'irradiation 2 à n'importe quel autre moment avant l'opération d'irradiation.

La poche d'irradiation 2 est irradiée sous agitation avec une lumière UV afin d'inactiver les éventuels pathogènes présents dans le fluide biologique.

Le bouchon amovible 27 fermant le deuxième orifice d'accès 5 est poussé à l'intérieur de la poche d'irradiation 2, puis le fluide biologique irradié est transféré dans la poche de stockage 8 pour être stocké jusqu'à 5 à 7 jours.

Après le transfert, la deuxième tubulure 9 est soudée et coupée à l'aide d'une soudeuse à main afin de séparer le récipient 2 du reste du système à poches 1.

Dans une réalisation, un échantillon du fluide biologique est envoyé dans la poche d'échantillonnage 11 pour analyse. Elle est séparée de la poche de stockage 8 en soudant et coupant la troisième tubulure 12, à l'aide d'une soudeuse à main.

### Exemples

### Exemple 1 : Impact de l'emballage et de différents éléments en PVC plastifié du système à poches sur la transmittance UV d'une gaine en EVA

Six configurations tests ont été réalisées et analysées :
- gaine EVA (contrôle) ;
- gaine EVA emballée dans un sachet constitué de deux films transparents composites de polyester (PET) et polyéthylène (PE) ;
- gaine EVA emballée dans un sachet constitué de deux feuilles de papier laqué ;
- poche de stockage en PVC plastifié par le BTHC disposée sur la gaine en EVA, et poche d'échantillonnage en PVC plastifié par le DEHP disposée à cheval sur la poche de stockage et la gaine en EVA, le tout emballé dans un sachet constitué d'un film transparent composite de PET et PE et d'une feuille en papier laqué;
- gaine en EVA protégée par un manchon polymère composite de PET et polypropylène (PP), une poche de stockage en PVC plastifié par le TOTM disposée sur la gaine EVA protégée, et une poche d'échantillonnage en PVC plastifié par le DEHP disposée à cheval sur la poche de stockage et la gaine EVA protégée, le tout emballé dans un sachet constitué d'un film transparent composite de PET et PE et d'une feuille en papier laqué.
- poche de stockage en PVC plastifié par le BTHC disposée sur la gaine en EVA, et poche d'échantillonnage en PVC plastifié par le DEHP disposée à cheval sur la poche de stockage et la gaine en EVA, le tout emballé dans un sachet constitué d'un film transparent composite de PET bi-orienté et polypropylène (PP) et d'une feuille en papier.

La gaine en EVA possède substantiellement les dimensions d'une poche d'irradiation.

Le manchon réalisé en polymère composite de PET et PP possède une épaisseur d'environ 55 µm, une perméabilité à l'oxygène inférieure à 91.0 cm3/m²/24h h à une température de 23 °C et une humidité relative de 50 % et une perméabilité à la vapeur d'eau inférieure à 5.0 cm³/m²/24h h à une température de 23 °C et une humidité relative de 50 %.

Les mesures de transmittance sont réalisées à l'aide d'un spectrophotomètre (Perkin Elmer Lamdbda650).

**[Tableaux1]**

| Transmittance UV-C (%) | Moyenne (%) T 0 | Moyenne (%) T 1 mois | Moyenne (%) T 3 mois |
|---|---|---|---|
| gaine EVA | 64,61 | 65,09 | 63,76 |
| gaine EVA | 64,61 | 54,90 | 52,70 |
| sachet PET/PE | | | |
| gaine EVA | 64,61 | 59,98 | 56,95 |
| sachet papier laqué | | | |
| gaine EVA | 64,61 | 61,08 | 57,17 |
| poche PVC/DEHP | | | |
| poche PVC/BTHC | | | |
| sachet PET/PP et papier laqué | | | |
| gaine EVA | 64,61 | 65,17 | 64,02 |
| manchon PET/PP | | | |
| poche PVC/DEHP | | | |
| poche PVC/BTHC | | | |
| sachet PET/PP et papier laqué | | | |
| gaine EVA | 64,61 | 63,75 | 63,22 |
| poche PVC/DEHP | | | |
| poche PVC/BTHC | | | |
| sachet PET biorienté/PP et papier | | | |

Ces résultats montrent l'impact négatif d'un sachet d'emballage particulier, à la fois par le film transparent et la feuille de papier laqué, sur la transmittance UV de la poche d'irradiation. Cet impact est renforcé par la présence des autres éléments du système à poches en PVC plastifié.

La présence du manchon protecteur permet de garantir l'absence de contamination de la poche d'irradiation en EVA par les plastifiants du PVC ou autres substances relargables de l'emballage et ainsi de maintenir une transmittance UV.

### Exemple 2 : Impact de la migration des plastifiants d'une feuille en PVC sur la transmittance UV d'une feuille en EVA.

Six configurations tests ont été réalisées en superposant des échantillons rectangulaires d'environ 60 cm² de feuilles de différents matériaux :
- deux échantillons de feuille en EVA ;
- deux échantillons de feuille en EVA et deux échantillons de feuille en PVC plastifié par le DEHP ;
- deux échantillons de feuille en EVA et deux échantillons de feuille en PVC plastifié par le BTHC
- deux échantillons de feuille en EVA et un échantillon de feuille en PVC plastifié par le DEHT ;
- deux échantillons de feuille en EVA et un échantillon de feuille en PVC plastifié par le TOTM ;
- deux échantillons de feuille en EVA et un échantillon de feuille en PVC plastifié par le DINCH.

La feuille en EVA en contact direct avec l'échantillon de feuille en PVC plastifié est identifiée « face A », la feuille en EVA placé sous la face A, est identifiée « face B ».

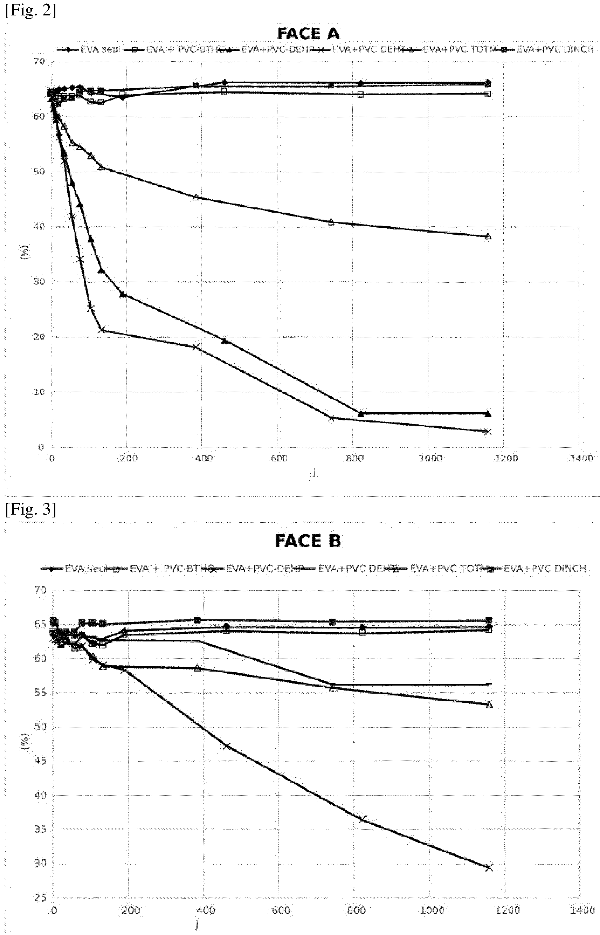

La transmittance UV des feuilles en EVA a été déterminée à différents temps entre 0 et 3 ans. Les résultats sont montrés sur les figures 2 et 3.

### 2.1 Impact du DEHP

On observe une forte chute de la transmittance UV de la feuille en EVA en contact direct avec un échantillon de feuille en PVC plastifié par le DEHP (face A). Dès le 14 ^{ème} jour de contact, la transmittance de la feuille en EVA est inférieure à 60%. Après deux ans, la transmittance UV de la feuille en EVA est inférieure à 10%.

Sur la face B, la perte de transmittance devient inférieure à 60% au bout de 100 jours.

La feuille en EVA ralentit mais n'empêche pas la migration de DEHP.

### 2.2 Impact du BTHC et du DINCH

Dans les conditions de l'étude, le contact de la feuille en EVA (face A et B) avec un échantillon de feuille en PVC plastifié par le BTHC ou le DINCH n'a aucune influence sur la transmittance de la feuille EVA tout au long des 3 ans.

### 2.3 Impact du DEHT

Le contact direct de l'échantillon de feuille en PVC plastifié par le DEHT avec la feuille en EVA (face A) provoque une perte de transmittance UV de la feuille en EVA encore plus marquée qu'avec la feuille en PVC plastifié par le DEHP.

L'impact de la feuille de PVC plastifié par le DEHT est moins marqué sur la face B, du fait de la présence de la face A qui ralentit la migration du DEHT.

### 2.4 Impact du TOTM

On observe une diminution de la transmittance UV de la feuille en EVA (face A) en contact direct avec un échantillon de feuille de PVC plastifié par le TOTM, connu pourtant pour peu migrer dans une matrice PVC mais absorbant les radiations UV, notamment UVC. Cependant, ce résultat est à prendre avec précaution car il représente la moyenne de trois tests, deux tests montrant une transmittance UV de la face en fin d'étude de 55-57% et un test une transmittance de moins de 2%. Les résultats sur la face B sont également disparates.

Ces études montrent que le DEHT est à éviter au même titre que le DEHP dans les systèmes à poches destinés à traiter par irradiation UV un fluide biologique.

### Exemple 3 : Impact de la migration des plastifiants du PVC en tubulure sur la transmittance UV d'une feuille en EVA.

Trois configurations tests ont été réalisées avec un échantillon rectangulaire de gaine en EVA de 15 cm x 18 cm seul ou en le superposant avec 60 cm de tubulure de différents matériaux, le tout emballé dans un sachet constitué d'un film transparent composite de PET bi-orienté et polypropylène et d'une feuille en papier :
- un échantillon de gaine en EVA (contrôle);
- un échantillon de gaine en EVA et un échantillon de tubulure de PVC plastifié par le DINCH;
- un échantillon de gaine en EVA et un échantillon de tubulure de PVC plastifié par le TOTM.

Comme pour la feuille de PVC plastifié par le DINCH, le contact d'une tubulure en PVC plastifié par le DINCH avec une gaine en EVA n'a aucune influence sur la transmittance UV de la gaine en EVA.

Le contact d'une tubulure en PVC plastifié par le TOTM avec une gaine en EVA entraine une baisse d'environ 5% de la transmittance UV de la gaine en EVA au bout de 90 jours. Cette baisse atteint plus de 10% au bout d'un an.

### Exemple 4 : Capacité de différentes matières à protéger la transmittance UV de la poche d'irradiation en EVA

Un même sachet d'emballage constitué d'un film transparent composite de PET et PE et d'une feuille en papier laqué est soudé de façon à former deux compartiments distincts. Chaque compartiment comprend un échantillon de gaine en EVA (15 cm x 15 cm) protégée par un manchon ouvert. De plus, dans le compartiment du haut, on intercale deux échantillons de PVC plastifié par le DEHP (15 cm x 15 cm) entre le sachet et la gaine en EVA protégée par le manchon.

Différents matériaux sont testés pour réaliser le manchon protecteur : film copolymère de PET, film copolymère de PET et PP ou film de PET non-tissé filé lié.

**[Tableaux2]**

| Transmittance UV-C (%) | PVC-DEH P | Moyenne (%) T0 | Moyenne (%) T 1 mois | Moyenne (%) 12 mois |
|---|---|---|---|---|
| Copolymère PET | Oui | 64,61 ± 0,66 | 64,68 ± 0,38 | 62,60 ± 1,16 |
| Copolymère PET | Non | 64,61± 0,66 | 64,83 ± 0,63 | 63,44 ± 0,54 |
| copolymère de PET et PP | Oui | 64,61± 0,66 | 64,07 ± 0,58 | 63,76 ± 0,67 |
| copolymère de PET et PP | Non | 64,61± 0,66 | 65,21 ± 0,41 | 64,05 ± 0,62 |
| PET non tissé | Oui | 64,61± 0,66 | 62,86 ± 0,49 | 49,42 ± 1,53 |
| PET non tissé | Non | 64,61± 0,66 | 59,72 ± 0,66 | 55,64 ± 0,59 |

Ces tests montrent que le film en copolymère de PET permet de protéger au moins à court terme la poche d'irradiation de la diffusion des plastifiants (DEHP) et autres substances contaminantes du sachet d'emballage.

Le manchon protecteur en copolymère de PET et PP procure une stabilité de la transmittance UV dans le temps.

Par contre, du fait de sa grande porosité, le manchon réalisé en non-tissé filé-lié de PET ne permet pas maintenir la transmittance UV de la gaine EVA.

## Revendications

1. Système à poches (1) pour le traitement par irradiation électromagnétique d'un fluide biologique, ledit système à poches comprenant au moins un élément constitué d'une poche d'irradiation (2) destinée à contenir le fluide biologique à irradier, ladite poche d'irradiation (2) étant réalisée dans un matériau perméable audit rayonnement électromagnétique, ledit système comprenant un ou plusieurs autres éléments choisis dans le groupe constitué par au moins une poche de stockage (8) et une tubulure (9), au moins un desdits autres éléments étant réalisé dans un matériau polymère formulé avec au moins un plastifiant, **caractérisé en ce qu'**il comprend en outre au moins un film protecteur (26a,26b) formant moyen barrière audit plastifiant et recouvrant ladite poche d'irradiation (2), ledit film protecteur (26a,26b) étant réalisé dans un matériau polymère barrière aux gaz ayant une perméabilité à l'oxygène inférieure ou égale à 100 cm³/m²/24 h à une température de 23 °C et une humidité relative de 50 %.

2. Système à poches selon la revendication 1, **caractérisé en ce que** le matériau polymère du film protecteur (26a,26b) est un matériau polymère barrière à la vapeur d'eau présentant une perméabilité à la vapeur d'eau inférieure ou égale à 10 g/m²/24 h à une température de 23 °C et une humidité relative de 50 %.

3. Système à poches selon l'une des revendications 1 ou 2, **caractérisé en ce que** le film protecteur (26a,26b) est transparent.

4. Système à poches selon l'une quelconque des revendications 1 à 3, **caractérisé en ce que** le film protecteur (26a,26b) comprend au moins une couche de polyester ou de polypropylène.

5. Système à poches selon l'une quelconque des revendications 1 à 4, **caractérisé en ce qu'**il comprend deux films protecteurs (26a,26b) agencés de part et d'autre de la poche d'irradiation (2).

6. Système à poches selon la revendication 5, **caractérisé en ce que** les deux films protecteurs (26a,26b) forment ensemble un manchon ayant au moins une extrémité ouverte dans lequel est disposée la poche d'irradiation (2).

7. Système à poches selon l'une quelconque des revendications 1 à 6, **caractérisé en ce que** le traitement par irradiation électromagnétique est un traitement par rayonnement UV et la poche d'irradiation est réalisée dans un matériau perméable à un rayonnement UV.

8. Système à poches selon l'une quelconque des revendications 1 à 7, **caractérisé en ce que** la poche d'irradiation (2) est exempte d'agent photosensible.

9. Système à poches selon l'une quelconque des revendications 1 à 8, **caractérisé en ce que** chacun desdits autres éléments est réalisé dans un ou des matériaux formulés sans phtalate ni téréphtalate.

10. Système à poches selon la revendication 9, **caractérisé en ce que** le ou lesdits autres éléments du système à poches comprennent un ou plusieurs éléments choisis dans le groupe constitué par une poche de stockage (8), une poche d'échantillonnage (11), une tubulure (6, 9, 12, 15), un connecteur (16), une pince (18a,18b, 18, 18d), un évent (7a, 7b, 17a, 17b) et leurs combinaisons.

11. Système à poches selon l'une des revendications 9 ou 10, **caractérisé en ce que** chacun desdits autres éléments dudit système à poches est réalisé dans un ou des matériaux autre que le polychlorure de vinyle plastifié par le phtalate de di-2-éthylhéxyle ou le téréphtalate de di-2-éthylhéxyle.

12. Système à poches selon l'une quelconque des revendications 9 à 11, **caractérisé en ce que** chacun desdits éléments du système à poches est réalisé soit en polychlorure de vinyle plastifié par un plastifiant choisi dans le groupe constitué par un ester d'acide cyclohexanedicarboxylique, un ester de citrate, un ester de trimellitate ou un mélange de ceux-ci, soit en un matériau autre que le polychlorure de vinyle plastifié.

13. Ensemble (20) pour le traitement par irradiation électromagnétique d'un fluide biologique comprenant un emballage (21) et un système à poches (1) selon l'une quelconque des revendications 1 à 12, **caractérisé en ce que** ledit système à poches (1) est confiné de façon stérile dans ledit emballage (21).

14. Ensemble selon la revendication 13, **caractérisé en ce que** l'emballage (21) est formé d'une feuille poreuse en non-tissé et d'une feuille en film transparent.

15. Utilisation d'un film (26a,26b) réalisé dans un matériau polymère barrière aux gaz ayant une perméabilité à l'oxygène inférieure ou égale à 100 cm³/m²/24 h à une température de 23°C et une humidité relative de 50 % comme moyen barrière aux plastifiants relargables du polychlorure de vinyle pour un système à poches selon l'une quelconque des revendications 1 à 14.

16. Utilisation d'un film selon la revendication 15, **caractérisée en ce que** le film est réalisé dans un matériau polymère barrière à la vapeur d'eau présentant une perméabilité à la vapeur d'eau inférieure ou égale à 10 g/ m²/24 h à une température de 23°C et une humidité relative de 50%.

## Patentansprüche

1. Beutelsystem (1) zur Behandlung einer biologischen Flüssigkeit durch elektromagnetische Bestrahlung, das Beutelsystem umfassend mindestens ein Element, das aus einer Bestrahlungsbeutel (2) besteht, die dazu bestimmt ist, die zu bestrahlende biologische Flüssigkeit zu enthalten, wobei der Bestrahlungsbeutel (2) aus einem Material gefertigt ist, das für die elektromagnetische Strahlung durchlässig ist, das System umfassend ein oder mehrere weitere Elemente, die ausgewählt sind aus der Gruppe, bestehend aus mindestens einem Speicherbeutel (8) und einem Schlauch (9), wobei mindestens eines der weiteren Elemente aus einem polymeren Material gefertigt ist, das mit mindestens einem Weichmacher formuliert ist, **dadurch gekennzeichnet, dass** es ferner mindestens einen Schutzfilm (26a,26b) umfasst, der eine Barriereeinrichtung für den Weichmacher bildet und den Bestrahlungsbeutel (2) bedeckt, wobei der Schutzfilm (26a,26b) aus einem Gasbarrierepolymermaterial gefertigt ist, das eine Sauerstoffdurchlässigkeit von weniger als oder gleich wie 100 cm³/m²/24 Std. bei einer Temperatur von 23 °C und einer relativen Feuchtigkeit von 50 % aufweist.

2. Beutelsystem nach Anspruch 1, **dadurch gekennzeichnet, dass** das Polymermaterial des Schutzfilms (26a,26b) ein Wasserdampfbarriere-Polymermaterial ist, das eine Wasserdampfdurchlässigkeit von weniger als oder gleich wie 10 g/m²/24 Std. bei einer Temperatur von 23 °C und einer relativen Feuchtigkeit von 50 % aufweist.

3. Beutelsystem nach einem der Ansprüche 1 oder 2, **dadurch gekennzeichnet, dass** der Schutzfilm (26a,26b) transparent ist.

4. Beutelsystem nach einem der Ansprüche 1 bis 3, **dadurch gekennzeichnet, dass** der Schutzfilm (26a,26b) mindestens eine Schicht aus Polyester oder Polypropylen umfasst.

5. Beutelsystem nach einem der Ansprüche 1 bis 4, **dadurch gekennzeichnet, dass** es zwei Schutzfilme (26a,26b) umfasst, die auf beiden Seiten des Bestrahlungsbeutels (2) ausgebildet sind.

6. Beutelsystem nach Anspruch 5, **dadurch gekennzeichnet, dass** die zwei Schutzfilme (26a,26b) zusammen eine Hülse bilden, die mindestens ein offenes Ende aufweist, in dem der Bestrahlungsbeutel (2) angeordnet ist.

7. Beutelsystem nach einem der Ansprüche 1 bis 6, **dadurch gekennzeichnet, dass** die Behandlung durch elektromagnetische Bestrahlung eine Behandlung durch UV-Strahlung ist und der Bestrahlungsbeutel aus einem Material gefertigt ist, das für UV-Strahlung durchlässig ist.

8. Beutelsystem nach einem der Ansprüche 1 bis 7, **dadurch gekennzeichnet, dass** der Bestrahlungsbeutel (2) frei von lichtempfindlichem Mittel ist.

9. Beutelsystem nach einem der Ansprüche 1 bis 8, **dadurch gekennzeichnet, dass** jedes der weiteren Elemente aus einem oder mehreren Materialien gefertigt ist, die ohne Phthalat und Terephthalat formuliert sind.

10. Beutelsystem nach Anspruch 9, **dadurch gekennzeichnet, dass** das oder die weiteren Elemente des Beutelsystems ein oder mehrere Elemente umfassen, die ausgewählt sind aus der Gruppe, bestehend aus einem Speicherbeutel (8), einem Probenahmebeutel (11), einem Schlauch (6, 9, 12, 15), einem Verbinder (16), einer Klemme (18a, 18b, 18, 18d), einer Entlüftung (7a, 7b, 17a, 17b) und Kombinationen davon.

11. Beutelsystem nach einem der Ansprüche 9 oder 10, **dadurch gekennzeichnet, dass** jedes der weiteren Elemente des Beutelsystems aus einem anderen Material oder Materialien als Polyvinylchlorid, das durch di-2-Ethylhexylphthalat oder di-2-Ethylhexylterephthalat plastifiziert ist, gefertigt ist.

12. Beutelsystem nach einem der Ansprüche 9 bis 11, **dadurch gekennzeichnet, dass** jedes der Elemente des Beutelsystems entweder aus Polyvinylchlorid, das durch einen Weichmacher plastifiziert ist, der ausgewählt ist aus der Gruppe, bestehend aus Cyclohexandicarbonsäureester, Citratester, Trimellitatester oder einem Gemisch davon, oder aus einem anderen Material als plastifiziertem Polyvinylchlorid gefertigt ist.

13. Anordnung (20) zur Behandlung einer biologischen Flüssigkeit durch elektromagnetische Bestrahlung, umfassend eine Verpackung (21) und ein Beutelsystem (1) nach einem der Ansprüche 1 bis 12, **dadurch gekennzeichnet, dass** das Beutelsystem (1) steril in der Verpackung (21) eingeschlossen ist.

14. Anordnung nach Anspruch 13, **dadurch gekennzeichnet, dass** die Verpackung (21) aus einer porösen Lage aus Vliesstoff und einer Lage aus durchsichtigem Film gebildet ist.

15. Verwendung eines Films (26a,26b), der aus einem Gasbarrierepolymermaterial gefertigt ist, das eine Sauerstoffdurchlässigkeit von weniger als oder gleich wie 100 cm³/m²/24 Std. bei einer Temperatur von 23 °C und einer relativen Feuchtigkeit von 50 % aufweist, als Barriereeinrichtung von Weichmachern, die aus dem Polyvinylchlorid freisetzbar sind, für ein Beutelsystem nach einem der Ansprüche 1 bis 14.

16. Verwendung eines Films nach Anspruch 15, **dadurch gekennzeichnet, dass** der Film aus einem Wasserdampfbarriere-Polymermaterial gefertigt ist, das eine Wasserdampfdurchlässigkeit von weniger als oder gleich wie 10 g/m²/24 Std. bei einer Temperatur von 23 °C und einer relativen Feuchtigkeit von 50 % besitzt.

## Claims

1. Bag system (1) for the treatment of a biological fluid by electromagnetic irradiation, said bag system comprising at least one element consisting of an irradiation bag (2) intended to contain the biological fluid to be irradiated, said irradiation bag (2) being made of a material that is permeable to said electromagnetic radiation, said system comprising one or more other elements selected from the group consisting of at least one storage bag (8) and a tubing (9), at least one of said other elements being made of a polymer material formulated with at least one plasticizer, **characterized in that** it further comprises at least one protective film (26a, 26b) forming a barrier means to said plasticizer and covering said irradiation bag (2), said protective film (26a, 26b) being made of a gas-barrier polymer material having a permeability to oxygen of less than or equal to 100 cm³ /m²/24 h at a temperature of 23°C and a relative humidity of 50%.

2. Bag system according to claim 1, **characterized in that** the polymer material of the protective film (26a, 26b) is a water vapor barrier polymer material with a permeability to water vapor of less than or equal to 10 g/m²/24 h at a temperature of 23°C and a relative humidity of 50%.

3. Bag system according to one of claims 1 or 2, **characterized in that** the protective film (26a, 26b) is transparent.

4. Bag system according to any of claims 1 to 3, **characterized in that** the protective film (26a, 26b) comprises at least one layer of polyester or polypropylene.

5. Bag system according to any one of claims 1 to 4, **characterized in that** it comprises two protective films (26a, 26b) arranged on either side of the irradiation bag (2).

6. Bag system according to claim 5, **characterized in that** the two protective films (26a, 26b) together form a sleeve with at least one open end in which the irradiation bag (2) is arranged.

7. Bag system according to any one of claims 1 to 6, **characterized in that** the electromagnetic irradiation treatment is a UV radiation treatment and the irradiation bag is made of a material permeable to UV radiation.

8. Bag system according to any one of claims 1 to 7, **characterized in that** the irradiation bag (2) is free of photosensitive agent.

9. Bag system according to any one of claims 1 to 8, **characterized in that** each of said other elements is made from a material or materials formulated without phthalate or terephthalate.

10. Bag system according to claim 9, **characterized in that** said other element(s) of the bag system comprise one or more elements selected from the group consisting of a storage bag (8), a sampling bag (11), tubing (6, 9, 12, 15), a connector (16), a clamp (18a, 18b, 18c, 18d), a vent (7a, 7b, 17a, 17b) and combinations thereof.

11. Bag system according to one of claims 9 or 10, **characterized in that** each of said other elements of said bag system is made of a material or materials other than polyvinyl chloride plasticized with di-2-ethylhexyl phthalate or di-2-ethylhexyl terephthalate.

12. Bag system according to any one of claims 9 to 11, **characterized in that** each of said elements of the bag system is made either of polyvinyl chloride plasticized with a plasticizer selected from the group consisting of a cyclohexanedicarboxylic acid ester, a citrate ester, a trimellitate ester or a mixture thereof, or of a material other than plasticized polyvinyl chloride.

13. Assembly (20) for the treatment of a biological fluid by electromagnetic irradiation comprising a package (21) and a bag system (1) according to any one of claims 1 to 12, **characterized in that** said bag system (1) is sterilely confined in said package (21).

14. Assembly according to claim 13, **characterized in that** the package (21) is formed of a porous non-woven sheet and of a transparent film sheet.

15. Use of a film (26a,26b) made of a gas-barrier polymer material having a permeability to oxygen of less than or equal to 100 cm³/m²/24 h at a temperature of 23°C and a relative humidity of 50% as a barrier to releasable plasticizers of polyvinyl chloride for a bag system according to any one of claims 1 to 14.

16. Use of a film according to claim 15, **characterized in that** the film is made of a water vapor-barrier polymer material having a permeability to water vapor of less than or equal to 10 g/m²/24 h at a temperature of 23°C and a relative humidity of 50%.
